# EUROPEAN PATENT APPLICATION

(11) **EP 1 970 049 A1**
(43) Date of publication of application: **17.09.2008**
(21) Application number: 07251094.4
(22) Date of filing: 15.03.2007
(51) Int. Cl.: A61K 9/10, A61K 31/59, A61P 17/06

(54) **Polyaphron topical composition with vitamin D and corticosteroid**

(71) Applicant: Drug Delivery Solutions Limited, Leatherhead, Surrey KT22 7RY (GB)
(72) Inventor: Wheeler, Derek Alfred, Leatherhead Surrey KT22 7RY (GB); Sindet-Pederson, Steen, Leatherhead Surrey KT22 7RY (GB); Steele, David Fraser, Leatherhead Surrey KT22 7RY (GB); Georgiou, Michelle, Leatherhead Surrey KT22 7RY (GB)
(74) Representative: Hayes, Adrian Chetwynd

(57) **Abstract**

A composition suitable for topical application comprising a continuous phase and at least one discontinuous phase, said composition comprising at least one polyaphron dispersion, at least one vitamin D or vitamin D analogue and at least one corticosteroid.

## Description

The present invention relates to a composition for topical application comprising at least one vitamin D or vitamin D analogue and at least one corticosteroid.

It is known for compositions comprising vitamin D or vitamin D analogues to be used for the treatment of a number of skin conditions.

For example, EP-B-474,517 discloses the use of compositions containing one or more 1α-hydroxylated-19-nor-vitamin D compounds with a triple bond in the side chain in the treatment of psoriasis.

US 4,871,723 discloses a process for treating psoriasis by topically applying a composition comprising vitamin D and a wax carrier. Specifically, US 4,871,723 discloses a composition comprising a) a pharmaceutically effective amount of an active-type vitamin D₃, b) a solvent selected from fatty acid esters, higher alcohols with 10 or more carbons and propylene carbonate and c) an oily carrier selected from white vaseline, yellow vaseline and liquid paraffin.

US 2005/002546 A1 discloses a pharmaceutical composition comprising an active vitamin D compound in emulsion pre-concentrate formulations, as well as emulsions and sub-micron droplet emulsions produced therefrom. In particular, the pharmaceutical compositions of US 2005/002546 A1 comprise
(a) a lipophilic phase component;
(b) one or more surfactants; and
(c) an active vitamin D compound.

The surfactant or surfactants are suitably present in an amount of 1% to 90% by weight based on the total weight of the composition, and preferably from about 5% to about 85% by weight based on the total weight of the composition.

Presently available compositions contain relatively high concentrations of vitamin D or vitamin D analogues and surfactants which often lead to skin irritation and worsening of psoriasis. For example, in 1996 the Food and Drug Administration of America required that the label included in Dovonex (calcipotriene)- a product containing 0.005% calcipotriol - be amended to indicate that approximately 25% of patients experienced skin irritation, and approximately 10% worsening of psoriasis. In addition, it has been reported that some patients treated with Dovonex have developed hypercalcaemia (see, for example, Hardman KA, Heath DA, Nelson HM Hypercalcaemia associated with calcipotriol (Dovonex) treatment. BMJ. 1993 Apr 3;306(6882):896-896).

Further, it is known in the prior art to treat a number of skin conditions by applying a combination of two or more pharmacologically active compounds. For example, in the treatment of psoriasis, it is possible to use a combination treatment involving a vitamin D analogue, such as calcipotriol, and a corticosteroid, where each of the active compounds is formulated in a separate preparation (for example in US-B-6,753,013).

Topical pharmaceutical compositions comprising a combination of a vitamin D analogue and a topical corticosteroid are challenging to manufacture. This is because these compounds are stable at different pH values.

For example, the calcipotriol requires a pH value above 8 for maximum stability, whereas corticosteroids, such as betamethasone (9-fluoro-11,17,21-trihydroxy-16-methylpregna-1,4-diene-3,20-dione), require pH values in the range 4 to 6 for maximum stability. It is therefore difficult to combine the two active components in a single formulation while maintaining good stability of the active compounds if water is present in the formulation.

US-B-6,753,013 discloses a pharmaceutical composition for dermal use, which contains at least one vitamin D or vitamin D analogue, at least one corticosteroid and a solvent selected to enable the two active components to coexist without significant degradation, despite their different stability profiles. The compositions, however, are wax-based and include wax or similar excipients, such as soft white paraffin and paraffin liquid. A disadvantage of this composition is that, in order to mix the vitamin D or vitamin D analogue and the corticosteroid into the wax, the wax must be heated to a temperature of 70°C. Such elevated temperatures may damage the drugs in the composition. Furthermore, wax based compositions tend to be rather oily, which after application leave a greasy film on the skin. This is undesirable and can lead to patient non-compliance.

A further disadvantage of the compositions of US-B-6,753,013 is that in order for the compositions to provide beneficial results in topical treatment, high levels of vitamin D or vitamin D analogue are required in order for sufficient vitamin D or vitamin D analogue to permeate the skin. This is because the compositions exhibit poor diffusion through skin. This is disadvantageous as vitamin D and vitamin D analogues are known skin irritants.

There is a need to formulate an improved composition suitable for topical application which addresses at least some of the problems of the prior art.

The present inventors have now developed a new composition comprising at least one vitamin D or vitamin D analogue and at least one corticosteroid. The present inventors have surprisingly found that such compositions have an enhanced dermal diffusion rate and/or improved stability compared to known compositions. Such compositions also have an appropriate viscosity such that they are useful for topical application.

Accordingly, the present invention provides a composition suitable for topical application comprising a continuous phase and at least one discontinuous phase, said composition comprising at least one polyaphron dispersion, at least one vitamin D or vitamin D analogue and at least one corticosteroid.

According to another aspect of the present invention there is provided a composition as described herein for use in the treatment of psoriasis.

According to another aspect of the present invention there is provided a composition as described herein for use in the manufacture of a medicament for the treatment of psoriasis.

According to a further aspect of the present invention there is provided a composition as described herein for use in a method of treatment of the human or animal body by therapy.

According to a further aspect there is provided a method of treatment or prophylaxis of psoriasis in a subject which comprises topically applying to a subject an effective amount of a composition as herein described.

In the following description, the meaning of the terms used are as follows: by hydrophilic phase or solvent is meant a liquid phase comprising water, comprising water together with other water-miscible liquids, or comprising a non-aqueous liquid which is miscible with water. By hydrophobic phase or solvent is meant a phase comprising pharmaceutically acceptable liquids such as oils that are immiscible or substantially immiscible with the hydrophilic phase. By immiscible liquids is meant that when mixed together, they separate to form two distinctly separate liquid phases sharing a well-defined interface. By substantially immiscible is meant that two liquids mixed as above having a well defined interface between two phases where each phase may nevertheless contain small quantities of dissolved molecules of the other phase.

According to another aspect of the present invention there is provided a method of making the composition as described herein comprising the following steps:
(i) providing a hydrophilic solvent, optionally comprising at least one vitamin D or vitamin D analogue, and/or at least one corticosteroid, and/or a surfactant;
(ii) providing a hydrophobic solvent optionally comprising at least one vitamin D or vitamin D analogue, and/or at least one corticosteroid, and/or a surfactant;
(iii)mixing the hydrophilic solvent with the hydrophobic solvent under suitable conditions to form the composition comprising at least one polyaphron dispersion, at least one vitamin D or vitamin D analogue and at least one corticosteroid.

According to a further aspect of the present invention there is provided a method of making the composition as described herein comprising the following steps:
preparing a first polyaphron dispersion comprising a vitamin D or vitamin D analogue;
preparing a second polyaphron dispersion comprising a corticosteroid;
and mixing together said first and second polyaphron dispersions to form the composition.

Advantageously, the compositions of the present invention have an enhanced dermal permeation of the active agent compared to known compositions, such as those disclosed in US-B-6,753,013. Thus, lower levels of active agents may be required in the compositions of the present invention in order to achieve beneficial treatment results. As a result of having lower levels of vitamin D and/or vitamin D analogues in the compositions of the present invention the likelihood of causing skin irritation and/or other side-effects is reduced. This may have the effect of increasing patient compliance with the dosage regime.

Advantageously, in the compositions of the present invention the vitamin D and/or vitamin D analogue and the corticosteroid can co-exist in a predominantly non-aqueous environment, having the appropriate and controllable viscosity due to the presence of polyaphron dispersions together with any gelling agents included in the composition. A further particular advantage is that the compositions have good long term stability even at elevated temperature (40°C). Furthermore, the compositions may contain water. This may be useful for dissolving water-soluble additives such as water-soluble preservatives, antioxidants, water-soluble permeation enhancers and the like.

A further advantage is that compositions of the present invention are typically manufactured at room temperature without the need to apply heat, making it less likely that actives will be damaged in the composition.

A further advantage of the present composition is that it feels less greasy in use compared to, for example, the compositions of US-B-6,753,013, making it more pleasant to apply and thereby increasing the likelihood of proper patient compliance.

A yet further advantage of the composition of the present invention is that it need not comprise a high level of surfactant. In high concentrations surfactants are known to cause skin irritation. It is therefore desirable to keep the surfactant level to a minimum when applied to skin, and in particular to damaged skin such as in the case of psoriasis. Preferably the compositions of the present invention comprise less than 4% by weight of surfactant, more preferably less than 3%, more preferably still less than 2% by weight of the total composition.

By polyaphron dispersion as used herein is meant a particular kind of hydrophilic liquid-in-hydrophobic liquid or hydrophobic liquid-in-hydrophilic liquid dispersion comprising (a) a hydrophilic liquid miscible phase, (b) a second hydrophobic phase being immiscible or substantially immiscible with the first phase and (c) one or more surfactants, wherein the dispersed or discontinuous phase is in the form of small (e.g. micron to sub-micron diameter, but more usually at least 1 micron diameter) droplets, and the whole having the following characteristics, which distinguish polyaphron dispersions from conventional or common emulsions and other dispersion types:
1. They are capable of existing in a stable form wherein the volume fraction of the dispersed phase (φᵢₚ) is greater than 0.7 and can be as high as 0.97. (φᵢₚ is the volume ratio of discontinuous to continuous phase expressed as a fraction).
2. The microscopic appearance of polyaphron dispersions where φᵢₚ is greater than 0.7 is that of an aggregate of individual droplets, pushed closely together into polyhedral shapes, resembling the appearance of a gas foam. In this form, the dispersion has gel-like properties and is referred to as a Gel Polyaphron Dispersion (GPD).
3. Stable polyaphron dispersions can be formed with a surfactant concentration less than 3% and more typically less than 2% by weight of the total composition.
4. Gel Polyaphron Dispersions (as described in 2 above) can be diluted to any extent by the addition of more continuous phase without the addition of more surfactant, when the gel-like properties disappear. Once φᵢₚ has been reduced to below 0.7, the individual droplets of internal phase become separated to take the form of spherical droplets, which remain stable and intact but which may nevertheless join together in loose associations and float to the top or sink to the bottom of the diluted dispersion (depending on the relative densities of the two phases). In this diluted form each droplet is referred to as a Colloidal Liquid Aphron (CLA). Simple shaking of the diluted dispersion instantly causes a homogeneous, stable dispersion of Colloidal Liquid Aphrons to re-form.
Each of the above characteristics and a combination of them clearly differentiate the polyaphron dispersions of the present invention from conventional emulsions and other dispersion types which do not have all of those characteristics. Polyaphron dispersions are disclosed in the following literature references by Sebba: "Biliquid Foams", J. Colloid and Interface Science, 40 (1972) 468-474 and "The Behaviour of Minute Oil Droplets Encapsulated in a Water Film", Colloid Polymer Sciences, 257 (1979) 392-396, Hicks "Investigating the Generation, Characterisation, and Structure of Biliquid Foams", PhD Thesis, University of Bristol, 2005, Crutchley "The Encapsulation of Oils and Oil Soluble Substances Within Polymer Films", PhD Thesis, The University of Leeds, 2006 and Lye and Stuckey, Colloid and Surfaces, 131 (1998) 119-136. Aphrons are also disclosed in US-A-4,486,333 and WO 97/32559.

Polyaphron dispersions are sometimes referred to as 'Biliquid Foams', 'High Internal Phase Emulsions (HIPEs)', 'High Internal Phase Ratio Emulsions (HIPREs)' and 'Gel Emulsions'. All such descriptions that refer to dispersions having the characteristics described above are polyaphron dispersions as used in the present invention.

The present invention will now be further described. In the following passages different aspects of the invention are defined in more detail. Each aspect so defined may be combined with any other aspect or aspects unless clearly indicated to the contrary. In particular any feature indicated as being preferred or advantageous may be combined with any other feature or features indicated as being preferred or advantageous.

As described above polyaphron dispersions comprise a continuous phase, a discontinuous phase and a surfactant. The discontinuous phase is preferably a substantially hydrophobic internal phase, commonly known as an oil internal phase. Preferably, the discontinuous phase comprises a pharmaceutically acceptable oil phase.

Examples of oils which may be used in the present invention include almond oil, babassu oil, blackcurrant seed oil, borage oil, canola oil, castor oil, coconut oil, cod liver oil, corn oil, cottonseed oil, evening primrose oil, fish oil, grapeseed oil, mustard seed oil, oat oil, olive oil, palm kernel oil, palm oil, peanut oil, rapeseed oil, safflower oil, sesame oil, shark liver oil, squalane, soybean oil, sunflower oil, walnut oil, wheat germ oil, hydrogenated castor oil, hydrogenated coconut oil, hydrogenated cottonseed oil, hydrogenated palm oil, hydrogenated soybean oil, partially hydrogenated soybean oil, hydrogenated vegetable oil, isopropyl myristate, isopropyl isostearate, isopropyl palmitate, modified triglycerides, caprylic/capric glycerides, fractionated triglycerides, glyceryl tricaprate, glyceryl tricaproate, glyceryl tricaprylate, glyceryl tricaprylate/caprate, glyceryl tricaprylate/caprate, glyceryl tricaprylate/caprate/laurate, glyceryl tricaprylate/caprate/linoleate, glyceryl tricaprylate/caprate/stearate, glyceryl trilaurate, glyceryl trilinoleate, glyceryl trilinolenate, glyceryl trioleate, glyceryl triundecanoate, linoleic glycerides, saturated polyglycolized glycerides, synthetic medium chain triglyceride containing primarily C₈-C₁₂ fatty acid chains, medium chain triglycerides, long chain triglycerides, modified triglycerides, fractionated triglycerides, and mixtures thereof.

Suitably the discontinuous phase comprises monoglycerides, diglycerides or triglycerides.

It will be understood that other suitable oils may be used in the present invention.

The discontinuous phase may, for example, confer an emollient, occlusive, moisturising, conditioning or other cosmetic or pharmaceutical benefit to the skin. It may also increase the viscosity of the composition and may confer solvency to the active or actives.

The composition may comprise at least 1 % by weight of the discontinuous phase, more preferably at least 10% by weight and most preferably at least 25% by weight based on the weight of the total composition.

The continuous or external phase of the polyaphron dispersion is preferably formed of a hydrophilic liquid, and is preferably completely or substantially non-aqueous. By substantially non-aqueous we mean that the total composition comprises less than 50% by weight of water, preferably less than 25%, more preferably less than 20%, more preferably still less than 10% by weight based on the total weight of the composition. The hydrophilic liquid itself preferably comprises less than 90 wt%, more preferably less than 50%, more preferably less than 10 wt% water based on the weight of the hydrophilic liquid. When any part of the continuous phase is aqueous, close control of the pH within suitable limits is essential as would be understood by those skilled in the art.

The continuous phase may comprise or consist essentially of a pharmaceutically acceptable liquid that is miscible or substantially miscible with water, preferably a compound of formula R₁-OH where R₁ is C₁-C₁₀ alkyl and/or a compound of formula HO-R₂-H where R₂ is - (C₂H₄)ₙ or (C₃H₆)ₙ where n is 1 to 100, preferably 1 to 25. R₁ and R₂ may be linear or branched. Preferably R₁ is C₁-C₄ alkyl. n is preferably 1 to 25. Preferably the continuous phase comprises propylene glycol, polyethylene glycol, glyercol, ethanol, or a mixture thereof. Where the continuous phase comprises polyethylene glycol or polypropylene glycol, the polyethylene or polypropylene glycol is preferably a polyethylene glycol which is liquid at room temperature (20°). The polyethylene glycol may, for example, contain from 1 to 12 ethylene or propylene oxide units and/or have a molecular weight of up to 600.

It will be understood that other suitable hydrophilic solvents may be used in the continuous phase of the polyaphrons.

The surfactant used in the present invention may be incorporated into either or both phases of the polyaphron dispersion. Suitable surfactants include an alkyl polyglycol ether, an alkyl polyglycol ester, an ethoxylated alcohol, a polyoxyethylene sorbitan fatty acid ester, a polyoxyethylene fatty acid ester, an ionic or non-ionic surfactant, a hydrogenated castor oil/polyoxyethylene glycol adduct containing from 25 to 60 ethoxy groups a castor oil/polyoxyethylene glycol adduct containing from 25 to 45 ethoxy groups, a sorbitan fatty acid ester (for example Span 20 or Span 80), a block copolymer of ethylene oxide and propylene oxide (for example Pluronic L121 or Pluronic F68), or a mixture thereof.

It will be understood that other suitable surfactants may be used.

Preferably the compositions of the present invention comprise less than 4% by weight of surfactant, more preferably less than 3%, more preferably still less than 2% by weight of the total composition.

The composition of the present invention may comprise at least one vitamin D or vitamin D analogue predominantly in the continuous phase, or predominantly in the discontinuous phase. Most preferably at least one vitamin D or vitamin D analogue is present predominantly in the discontinuous phase.

The vitamin D analogue employed in the composition of the present invention may, for example, be calcipotriol, seocalcitol, calcitriol, calcipotriol monohydrate, tacalcitol, maxacalcitol, paricalcitol, falecalcitriol, 1α,24S-dihydroxy-vitamin D2, 1(S),3(R)-dihydroxy-20(R)-[((3-(2-hydroxy-2-propyl)-phenyl)-methoxy)-methyl]-9,10-seco-pregna-5(Z),7(E),10(19)-triene, or a mixture thereof. More preferably, the vitamin D anolgue is calcipotriol, calcitriol, tacalcitol, maxacalcitol, 1α,24S-dihydroxy-vitamin D2, 1(S),3(R)-dihydroxy-20(R)-[((3-(2-hydroxy-2-propyl)-phenyl)-methoxy)-methyl]-9,10-seco-pregna-5(Z),7(E),10(19)-triene, or a mixture thereof. Most preferably, the vitamin D analogues are calcipotriol and calcipotriol monohydrate. Other examples of suitable vitamin D analogues are described in US-B-6,753,013.

Synthetic vitamin D analogues are preferred in the compositions of the present invention over naturally occurring vitamin D or vitamin D derivatives, since the therapeutic effects of the latter may be less selective for the treatment of skin diseases, such as psoriasis.

The composition of the present invention may comprise from 0.0001 to 0.05% by weight of vitamin D or vitamin D analogue, preferably from 0.001 to 0.01% by weight and more preferably from 0.0025 to 0.005% by weight of the total composition.

The corticosteroid may be predominantly in the continuous phase, or predominantly in the discontinuous phase. Preferably, the corticosteroid is predominantly in the discontinuous phase. More preferably, both the corticosteroid and the vitamin D or a vitamin D analogue are predominantly in the discontinuous phase. The range of weight ratios of corticosteroid to vitamin D or vitamin D analogue is preferably 4:1 to 50:1 and more preferably 8:1 to 20:1 and most preferably 9:1 to 11:1.

Preferably, the corticosteroid is selected from one or more of Betamethasone (9 -fluoro-11 ,17 ,21-trihydroxy-16-methylpregna-1,4-diene-3,20-dione) or esters thereof such as the 21-acetate, 17-adamantoate, 17-benzoate, 17-valerate, and 17,21-dipropionate; Alclomethasone or esters thereof such as the 17,21-dipropionate; Clobetasole or esters thereof such as the propionate; Clobetasone or esters thereof such as the 17-butyrate; Desoximetasone; Diflucortolon or esters thereof, Diaflorasone or esters thereof such as the 17,21-diacetate; Fluocinonid; Flumetasone or esters thereof such as the 21-pivalate; Fluocinolone or ethers thereof such as the acetonide; Fluticasone or ester thereof such as the 17-propionate; Fluprednidene or esters thereof such as the 21-acetate; Halcinonide; Hydrocortisone or esters thereof such as the 17-butyrate; Mometasone or esters thereof such as the 17-(2-furoate); and Triamcinolon or ethers or esters thereof such as the acetonide. More preferably the corticosteroid is selected from one or more of Betamethasone or esters thereof such as the 17-valerate or the 17,21-dipropionate; Clobetasole or esters thereof such as the propionate; Triamcinolon or ethers or esters thereof such as the acetonide or the acetonide-21-N-benzoyl-2-methyl- - alaninate or the acetonide-21-(3,3-dimethylbutyrate); or Hydrocortisone or esters thereof such as the 17-butyrate. Most preferably, the corticosteroid is betamethasone 17,21-dipropionate.

The composition of the present invention preferably comprises from 0.001 to 1.0% by weight of corticosteroid, more preferably from 0.01% to 0.075% by weight and more preferably still from 0.025 to 0.05% by weight of the total composition.

The composition of the present invention may further comprise a gelling agent and/or a rheology modifying agent, such as a viscosity modifier.

The gelling agent may, for example, be selected from alginate gums or their salts, guar gum, locust bean gum, xanthan gum, gum acacia, gelatin, hydroxymethyl-cellulose hydroxyethylcellulose, hydroxypropyl-cellulose, carboxymethylcellulose or its salts, bentonites, magnesium aluminium silicates, "Carbomers" (salts of cross-linked polymers of acrylic acid), or glyceryl polymethacrylates or their dispersions in glycols. It will be understood that other suitable gelling agents may be used.

Preferably, the composition of the present invention comprises from 0.05 to 5.0% by weight of a gelling agent, preferably from 0.1 to 2.0% by weight and more preferably from 0.2 to 1.0% by weight of the total composition.

The composition of the present invention may be used in a method of treatment of the human or animal body by therapy. Further, the composition of the present invention may be used in the treatment of psoriasis. Also the composition of the present invention may be used in the manufacture of a medicament for treatment of psoriasis.

The compositions of the present invention may also contain other additives such as preservatives (for instance to prevent microbiological spoilage), buffering agents (for the control of pH and to avoid instability and damage to the skin's acid mantle), antioxidants and permeation enhancers. These additives may be included in the continuous or the discontinuous phase of the polyaphron dispersion.

It will be understood that the inclusion of these additives will be at the levels and with the type of materials which are found to be effective and useful. Care needs to be taken in the choice and amount of these additives to prevent compromise to the other performance advantages of the present invention.

In one embodiment of the present invention, the vitamin D or vitamin D analogue of the present invention is dispersed and/or dissolved in the discontinuous phase of a first polyaphron dispersion. The corticosteroid is dispersed and/or dissolved in the discontinuous phase of a second polyaphron dispersion. The first and the second polyaphron dispersions are then mixed together to form the composition of the present invention. Optionally, a third or further polyaphron dispersion may also be present in the composition of the present invention. The third or further polyaphron dispersion may, for example, comprise agents such as emollient oils (to improve in use 'feel') or sunscreens. Preferably these agents will be present in the discontinuous phase of the polyaphron dispersions.

In one embodiment of the present invention, the vitamin D analogue is calcipotriol or calcipotriol monohydrate.

In a particularly preferred composition the discontinuous phase is a caprylic/capric triglyceride, the continuous phase is demineralised water, the vitamin D analogue is calcipotriol and the corticosteroid is betamethasone dipropionate.

Accordingly to one aspect of the present invention, there is provided a method of making the composition as described herein comprising the following steps:
(i) providing a hydrophilic solvent, optionally comprising at least one vitamin D or vitamin D analogue, and/or at least one corticosteroid, and/or a surfactant;
(ii) providing a hydrophobic solvent optionally comprising at least one vitamin D or vitamin D analogue, and/or at least one corticosteroid, and/or a surfactant;
(iii) mixing the hydrophilic with the hydrphobic solvent under suitable conditions to form the composition comprising at least one polyaphron dispersion, at least one vitamin D or vitamin D analogue and at least one corticosteroid.

Suitable methods for preparing polyaphron dispersions are described in US-A-4486333. It will be understood by those skilled in the art that other manufacturing methods may be used, as appropriate.

Accordingly to another aspect of the present invention, there is provided a method of making the composition as described herein comprising the following steps:
preparing a first polyaphron dispersion comprising a vitamin D or vitamin D analogue;
preparing a second polyaphron dispersion comprising a corticosteroid;
and mixing together said first and second polyaphron dispersions to form the composition.

The method may further comprise:
preparing a third or further polyaphron dispersion comprising an active agent, such as a sunscreening agent, antioxidant or emollient oils;
and mixing said third or further polyaphron with said first and second polyaphron dispersions to form the composition.

The following Examples further illustrate the present invention.

### EXAMPLE 1

Three gel polyaphron dispersions of the following compositions were prepared by the following method.

| ***Gel POLYAPHRON DISPERSION 1*** | |
|---|---|
| ***Oil Phase*** | **%** |
| Calcipotriol solution* (0.0695%) in Caprylic/Capric Triglyceride (Mygliol 812 - Condea) | 89.10 |
| Laureth-4 (Volpo L4 - Croda) | 0.90 |
| | |

| ***Aqueous Phase*** | |
|---|---|
| Poloxamer 188 (Pluronic F68 - BASF) | 1.00 |
| Demineralised Water | 9.00 |

| ***Gel POLYAPHRON DISPERSION 2*** | |
|---|---|
| ***Oil Phase*** | **%** |
| Betamethasone Dipropionate solution** (0.3465%) in Caprylic/Capric Triglyceride (Mygliol 812 - Condea) | 89.10 |
| Laureth-4 (Volpo L4 - Croda) | 0.90 |
| | |

| *Aqueous Phase* | |
|---|---|
| Poloxamer 188 (Pluronic F68 - BASF) | 1.00 |
| Demineralised Water | 9.00 |

| ***Gel POLYAPHRON DISPERSION 3*** | |
|---|---|
| ***Oil Phase*** | % w/w |
| Squalane (Olive-derived - A & E Connock) | 9.00 |
| Dimethicone (Q7-1920, 100 CP - Dow Corning) | 44.10 |
| Cyclomethicone (STb5NF - Dow Corning) | 36.00 |
| Laureth-4 (Volpo L4 - Croda) | 0.90 |
| | |

| *Aqueous Phase* | |
|---|---|
| Poloxamer 188 (Pluronic F68 - BASF) | 1.00 |
| Demineralised Water | 9.00 |

| ***FINAL PRODUCT*** | % w/w |
|---|---|
| *GEL POLYAPHRON DISPERSION 1* | 7.20 |
| *GEL POLYAPHRON DISPERSION* 2 | 18.60 |
| *GEL POLYAPHRON DISPERSION* 3 | 74.20 |

| | |
|---|---|
| *The Calcipotriol content in the final formulation is 50µg/g. **The betamethasone dipropionate content in the final formulation is 643µg/g (equivalent to 500µg/g of betamethasone). | |

### Manufacturing Method

Three polyaphron dispersions were made individually by the following method:
A low form, 250ml laboratory beaker (internal diameter 6.5cm) was charged with sufficient aqueous (continuous) phase to make 30 g of gel polyaphron. This was stirred at 200 rpm with a four-bladed impeller having a diameter of 6.0 cm whilst adding the oil (discontinuous) phase drop wise from a Pasteur pipette. The rate of addition at the start of the process was slow (approximately one drop every 7 seconds) but was speeded up once 10% of the oil phase had been added so that the total time to make the gel polyaphron was approximately 20 minutes.

Prior to the manufacture of each gel polyaphron dispersion, any active was dissolved in the appropriate phase by gentle stirring overnight with a magnetic stirrer at room temperature in a covered beaker.

To form the final product, the three individual polyaphron dispersions were mixed together.

### Stability Measurements

Stability measurements made using the method outlined below.

The calcipotriol and betamethasone were extracted from the composition of Example 1 into isopropanol and assayed by HPLC under the conditions given below.

### HPLC conditions:

Column:NovaPakC18, 4µ particle size, 3.9 x 150mm column (Waters)
Mobile phase: 47% acetronitrile in water.
Flow rate: 1ml/minute.
Column Temperature: 25°C.
Retention time for calcipotriol was 6.8 minutes
Retention tine for betamethasone was 9.9 minutes.

The inventors observed that after 2 months storage at 40°C, the levels of calcipotriol and betamethasone were 102% ±3% and 99% ±1% of the original levels, respectively.

### Example 2

| ***FINAL PRODUCT*** | **% w/w** |
|---|---|
| *GEL POLYAPHRON DISPERSION 1* | 7.20 |
| *GEL POLYAPHRON DISPERSION* 2 | 18.60 |
| *GEL POLYAPHRON DISPERSION* 3 | 69.20 |
| *Propylene Glycol* | 5.00 |

### Manufacturing Method

The method used was precisely as described for Example 1 above except that propylene glycol was added to the final mixture of the three gel polyaphron dispersions, 1, 2 and 3 and then mixed in by simple mixing until the product was a homogeneous mixture of the polyaphron dispersions.

### Stability Measurements

Stability was tested as in Example 1

The inventors observed that after 3 weeks storage at 40°C the levels of calcipotriol and betamethasone were 98.6±1.8% and 100.5+1.1% of the original levels respectively. Reference is made to Simonsen et al, Drug Development and Industrial Pharmacy, 30(10) (2004) 1095-1102, wherein the authors state that the inclusion of propylene glycol to a product containing both calcipotriol and betamethasone causes rapid decomposition of one or other of the actives depending upon pH, although propylene glycol is a very good flux rate enhancer. They concluded that it was not possible to include propylene glycol in their formulated product. This Example demonstrates that the inventors of the present invention have overcome this problem. Example 2, table 2 in US-B-6,753,013 illustates the degradation of calcipotriol in a product comprising calcipotriol, betamethasone dipropionate and propylene glycol.

### Example 3

The compositions of Examples 1 and 2 were tested for steady state of flux rate (measured over 8 hours) through silicone membranes in Franz diffusion cells independently for the calcipotriol and betamethasone. This is a recognised in vitro test correlating to permeation through the skin. For comparison the commercial product Dovobet was also tested.

| **Compositions** | **Calcipotriol Flux Rate ng cm⁻²hr⁻¹** | **Ratio of rate to that of Dovobet** |
|---|---|---|
| Example 1 composition | 94.01 | 2.6 |
| Example 2 Composition | 69.78 | 1.9 |
| Dovobet | 36.38 | 1.0 |

| **Compositions** | **Betamethasone Flux Rate ng cm⁻²hr⁻¹** | **Ratio of rate to that of Dovobet** |
|---|---|---|
| Example 1 Composition | 1236.88 | 2.5 |
| Example 2 Composition | 907.00 | 1.8 |
| Dovobet | 494.64 | 1.0 |

## Claims

1. A composition suitable for topical application comprising a continuous phase and at least one discontinuous phase, said composition comprising at least one polyaphron dispersion, at least one vitamin D or vitamin D analogue and at least one corticosteroid.

2. A composition according to claim 1 wherein the corticosteroid is betamethasone, clobetasol, clobetasone, desoximethasone, diflucortolon, difluorasone, fluocinoid, flumethasone, fluocinolon, fluticasone, fluprednidene, halcinonide, hydrocortisone, momethasone, triamcinolon, and their esters or a mixture thereof.

3. A composition according to claim 2 wherein the corticosteroid is betamethasone or an ester thereof.

4. A composition according to any one of the preceding claims wherein the vitamin D or vitamin D analogue is vitamin D, calcipotriol, seocalcitol, calcitriol, tacalcitol, maxacalcitol, paricalcitol, falecalcitriol, 1α,24S-dihydroxy-vitamin D2, 1(S),3(R)-dihydroxy-20(R)-[((3-(2-hydroxy-2-propyl)-phenyl)-methoxy)-methyl]-9,10-seco-pregna-5(Z),7(E),10(19)-triene, or a mixture thereof.

5. A composition according to claim 4 wherein the vitamin D or vitamin D analogue is calcipotriol.

6. A composition according to any one of the preceding claims wherein the discontinuous phase comprises an oil.

7. A composition according to claim 6 wherein the oil comprises a monoglyceride, diglyceride or triglyceride or a mixture thereof.

8. A composition according to any one of the preceding claims wherein the continuous phase comprises from 0 to 50%, by weight of water based on the total weight of the composition.

9. A composition according to claim 8 wherein the continuous phase comprises less than 20% by weight of water, based on the total weight of the composition.

10. A composition according to claim 8 wherein the continuous phase comprises less than 10% by weight of water, based on the total weight of the compound.

11. A composition according to claim 8 wherein the continuous phase is non-aqueous.

12. A composition according to any one of the preceding claims wherein the continuous phase comprises a compound of formula R₁-OH where R₁ is C₁-C₁₀ alkyl and/or a compound of formula HO-R₂-H where R₂ is (C₂H₄)ₙ or (C₃H₆)ₙ where n is 1 to 100.

13. A composition according to claim 11 wherein the continuous phase comprises propylene glycol, glycerol, ethanol, or a mixture thereof.

14. A composition according to any one of the preceding claims wherein the corticosteroid is predominantly in the discontinuous phase.

15. A composition according to any one of the preceding claims wherein the vitamin D or vitamin D analogue is predominantly in the discontinuous phase.

16. A composition according to any one of the preceding claims further comprising a gelling agent.

17. A composition according to any one of the preceding claims further comprising a permeation enhancer.

18. A composition as defined in anyone of claims 1 to 17 for use in a method of treatment of the human or animal body by therapy.

19. A composition as defined in any one of claims 1 to 17 for use in the treatment of psoriasis.

20. Use of a composition as defined in any one of claims 1 to 17 in the manufacture of a medicament for treatment of psoriasis.

21. A method of making the composition as defined in any one of claims 1 to 17 comprising the following steps:
(i) providing a hydrophilic solvent, optionally comprising at least one vitamin D or a vitamin D analogue, and/or at least one corticosteroid, and/or a surfactant;
(ii) providing a hydrophobic solvent optionally comprising at least one vitamin D or a vitamin D analogue, and/or at least one corticosteroid, and/or a surfactant;
(iii)mixing the hydrophilic solvent with the hydrophobic solvent under suitable conditions to form the composition comprising at least one polyaphron dispersion, at least one vitamin D or vitamin D analogue and at least one corticosteroid.

22. A method of making the composition as defined in any one of claims 1 to 17 comprising the following steps:
preparing a first polyaphron dispersion comprising a vitamin D or vitamin D analogue;
preparing a second polyaphron dispersion comprising a corticosteroid;
and mixing together said first and second polyaphron dispersions to form the composition.

23. The method according to claim 21 or 22 wherein the vitamin D or vitamin D analogue is predominantly in the discontinuous phase.

24. The method according to claim 21, 22 or 23 wherein the corticosteroid is predominantly in the discontinuous phase.

25. The method according to any one of claims 21 to 24 further comprising
preparing a third or further polyaphron dispersion comprising an active agent;
and mixing said third or further polyaphron dispersion with said first and second polyaphron dispersions.
